(19) Europäisches Patentamt
European Patent Office
Office européen des brevets

(11) Publication number: **0 166 353 B1**

# (12) EUROPEAN PATENT SPECIFICATION

(45) Date of publication of patent specification: **18.03.92**

(51) Int. Cl.⁵: **C07D 223/16**, C07D 215/38, C07D 225/06, A61K 31/55

(21) Application number: **85107498.9**

(22) Date of filing: **20.06.85**

(54) **Benzofused lactams useful as antihypertensive agents and as cholecystokinin antagonists.**

(30) Priority: **26.06.84 US 624855**

(43) Date of publication of application:
**02.01.86 Bulletin 86/01**

(45) Publication of the grant of the patent:
**18.03.92 Bulletin 92/12**

(84) Designated Contracting States:
**CH DE FR GB IT LI NL**

(56) References cited:
**EP-A- 0 072 352**

(73) Proprietor: **MERCK & CO. INC.**
**126, East Lincoln Avenue P.O. Box 2000**
**Rahway New Jersey 07065-0900(US)**

(72) Inventor: **Parsons, William H.**
**2171 Oliver Street**
**Rahway New Jersey 07065(US)**

(74) Representative: **Abitz, Walter, Dr.-Ing. et al**
**Abitz, Morf, Gritschneder, Freiherr von Wittgenstein Postfach 86 01 09**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### BACKGROUND OF THE INVENTION

The present invention is concerned with novel benzofused lactams which are effective inhibitors of angiotensin I converting enzyme and effective antagonists of cholecystokinin. These novel compounds are, consequently, combined with pharmaceutically acceptable carriers to form pharmaceutical compositions of the present invention and are used in a method of treating hypertension, gastrointestinal disorders, central nervous system disorders, or regulating appetite in humans.

Angiotensin II, a powerful vasoconstrictor hormonal peptide, is formed from the inactive angiotensin I by the action of angiotensin converting enzyme (ACE). Recently, potent inhibitors of ACE have been reported which are capable of lowering the blood pressure in hypertensive patients. The novel benzofused lactams of the present invention are also potent inhibitors of ACE.

Cholecystokinin (CCK) is a neuropeptide composed of thirty-three aminoacids. See: Mutt and Jorpes, Biochem. J. 125 678 (1971). The carboxyl terminal octapeptide (CCK-8) also occurs naturally and is fully active. CCK exists in both gastrointestinal tissue and the central nervous system. V. Mutt, Gastrointestinal Hormones, G. B. J. Glass, Ed., Raven Press, N.Y., (1980) p. 169. CCK is believed to play an important role in appetite regulation and CCK may be a physiological satiety hormone. G. P. Smith, Eating and Its Disorders, A. J. Stunkard and E. Stellar, Eds, Raven Press, New York, 1984, p. 67.

Among additional effects of CCK are stimulation of colonic motility, stimulation of gall bladder contraction, stimulation of pancreatic enzyme secretion, and inhibition of gastric emptying. CCK reportedly co-exists with dopamine in certain mid-brain neurons and thus may also play a role in the functioning of dopaminergic systems in the brain as well as serving as a neurotransmitter in its own right. See: A. J. Prange et al., "Peptides in the Central Nervous System", Ann. Repts. Med. Chem. 17 31, 33 (1982) and references cited therein; J. A. Williams, Biomed. Res. 3 107 (1982); and J. E. Morley, Life Sci. 30, 479 (1982).

CCK antagonists are useful in the treatment and prevention of CCK-related disorders of the gastrointestinal, central nervous and appetite regulatory systems of animals, especially humans. Three distinct chemical classes of CCK receptor antagonists have been reported. One class comprises derivatives of cyclic nucleotides; detailed structure-function studies have demonstrated that of the various members of this class, dibutyryl cyclic GMP is the most potent. See; N. Barlos et al., Am. J. Physiol., 242, G161 (1982) and P. Robberecht et al., Mol. Pharmacol., 17, 268 (1980). The second class comprises peptide antagonists which are C-terminal fragments and analogs of CCK. Recent structure-function studies have shown that both shorter C-terminal fragments of CCK (Boc-Met-Asp-Phe-$NH_2$, Met-Asp-Phe-$NH_2$) as well as longer CCK fragments (Cbz-Tyr($SO_3H$)-Met-Gly-Trp-Met-Asp-$NH_2$) can function as CCK antagonists. See: R. T. Jensen et al., Biochim. Biophys. Acta., 757, 250 (1983) and M. Spanarkel et al., J. Biol. Chem., 258, 6746 (1983). The third class of CCK receptor antagonists comprises the amino acid derivatives; proglumide, a derivative of glutaramic acid, and the N-acyl tryptophans including para-chlorobenzoyl-L-tryptophan (benzotript). See W. F. Hahne et al., Proc. Natl. Acad. Sci. U.S.A., 78, 6304 (1981) and R. T. Jensen et al., Biochem. Biophys. Acta., 761, 269 (1983). All of these compounds are relatively weak antagonists of CCK ($IC_{50}$: $10^{-4}$-$10^{-6}$ M).

EP-A-0 072 352 describes 3-amino-[1]-benzazepin-2-on-1-alkanoic acids which are said to be effective as angiotensin converting enzyme (ACE) inhibitors and can be used as antihypertensive agents. There is no indication that these compounds are effective cholecystokinin antagonists.

### SUMMARY OF THE INVENTION

It has now been found that the compounds of this invention are inhibitors of angiotensin converting enzyme (ACE) and certain of these compounds are antagonists of cholecystokinin (CCK). Thus, these compounds are useful in the treatment of hypertension and some are useful in the treatment and prevention of CCK-related disorders of the gastrointestinal, central nervous and appetite regulatory systems of animals, especially humans.

### DETAILED DESCRIPTION OF THE INVENTION

In its broadest aspect, the present invention relates to novel benzofused lactams of the formula:

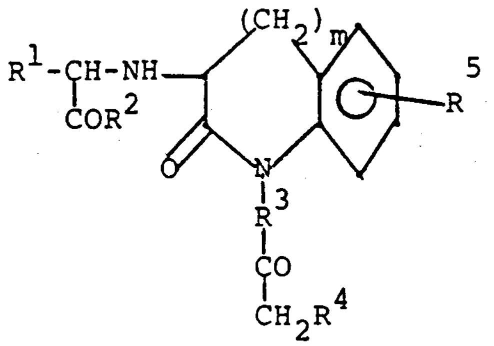

I

wherein:

$R^1$ is hydrogen;

hydrocarbon of from 1 to 12 carbon atoms which include branched and unsaturated alkyl groups;

$C_3$-$C_{10}$cycloalkyl;

substituted $C_{1-6}$alkyl wherein the substituent can be halo, hydroxy, carboxy, $C_{1-6}$alkylthio, $C_{1-6}$alkoxy, $C_{1-6}$alkoxycarbonyl, carboxamido, $C_{1-6}$aralkoxycarbonyl wherein aryl is phenyl, naphthyl or biphenyl, amino, $C_{1-6}$alkylamino, $C_{1-6}$dialkylamino, or $C_{1-6}$alkanoylamino and aroylamino wherein aryl is phenyl, naphthyl or biphenyl;

substituted $C_{1-6}$alkyl having the formula $R^1_A$ $(CH_2)_p$-Q-$(CH_2)_q$ wherein p is 0-2, q is 1-3, $R^1_A$ is aryl or heteroaryl wherein aryl is phenyl, naphthyl or biphenyl and heteroaryl is a 5- or 6-membered aromatic ring containing from 1 to 3 heteroatoms selected from the group consisting of nitrogen, oxygen and sulfur and bicyclic groups in which the above-mentioned 5- or 6-membered aromatic rings are fused to a benzene ring and which aryl or heteroaryl group is optionally substituted by amino, $C_{1-6}$dialkylamino, $C_{1-6}$alkylamino, hydroxy, hydroxy-$C_{1-6}$alkyl, amino-$C_{1-6}$alkyl, trihalo-$C_{1-6}$alkyl, cyano, nitro, sulfonamido, aroyl wherein aryl is as defined above, $C_{1-6}$alkyl, halo, dihalo, and $C_{1-6}$alkoxy, and Q is O, S, N-$R^1_B$ , CON$R^1_C$ , N$R^1_C$ CO, CH = CH

wherein $R^1_B$

is hydrogen, $C_{1-6}$alkyl, aryl as defined above, aralkyl wherein the alkyl portion is $C_{1-6}$alkyl and the aryl portion is as defined above, $C_{1-6}$-alkanoyl, or aroyl wherein aryl is as defined above, and $R^1_C$ is hydrogen, or $C_{1-6}$alkyl;

aryl as defined above;

substituted aryl as defined above wherein the substituent is $C_{1-6}$alkyl, amino-$C_{1-6}$alkyl, $C_{1-6}$alkoxy, aryloxy wherein aryl is as defined above, aroyl wherein aryl is as defined above, hydroxy, halo, or dihalo; substituted or unsubstituted aralkyl or substituted or unsubstituted heteroaralkyl wherein the alkyl portion is $C_{1-6}$alkyl and the aryl portion and heteroaryl portion are as defined above and which include branched $C_{1-6}$alkyl groups wherein the $C_{1-6}$alkyl groups can be substituted by amino, $C_{1-6}$alkanoylamino and aroylamino wherein aryl is as defined above, or hydroxyl and the aryl and heteroaryl groups can be substituted by halo, dihalo, $C_{1-6}$alkyl, hydroxy, $C_{1-6}$alkoxy, aryloxy wherein aryl is as defined above, aroyl wherein aryl is as defined above, arylthio wherein aryl is as defined above, amino, amino-$C_{1-6}$alkyl, $C_{1-6}$alkanoylamino, aroylamino wherein aryl is as defined above, $C_{1-6}$dialkylamino, $C_{1-6}$alkylamino, hydroxy-$C_{1-6}$alkyl, trihalo-$C_{1-6}$alkyl, nitro, cyano, or sulfonamido;

$R^2$ is hydroxy; $C_{1-6}$alkoxy;

$C_{2-6}$alkenyloxy; aryl-$C_{1-6}$alkoxy wherein aryl is as defined above; $C_{1-6}$alkanoyl-$C_{1-6}$alkoxy; amino; $C_{1-6}$alkylamino; di-$C_{1-6}$alkylamino; aryl-$C_{1-6}$alkylamino wherein aryl is as defined above; arylamino wherein aryl is as defined above; carboxy-$C_{1-6}$-alkylamino; carboxamido-$C_{1-6}$alkylamino; or aryloxy, or mono- or disubstituted aryloxy wherein aryl is as defined above and wherein the substituents are halo;

m is 1-3;

$R^3$ is $C_{1-6}$alkyl or substituted $C_{1-6}$alkyl wherein the substituents are hydrogen, $C_{1-6}$alkyl, or aryl as defined above;

$R^4$ is hydroxy; $C_{1-6}$alkanoyloxy; aryl-$C_{1-6}$alkanoyloxy wherein aryl is as defined above;

$R^5$ is hydrogen; halo; hydroxy; $C_{1-6}$alkyl; or $C_{1-6}$alkoxy; and
a pharmaceutically acceptable salt thereof.

The $C_{1-6}$alkyl substituents recited above represent, except where otherwise indicated, any of the variables of straight, branched, and unsaturated chain hydrocarbon radicals such as methyl, ethyl, n-propyl, i-propyl, n-butyl, i-butyl, t-butyl, n-pentyl, i-pentyl, n-hexyl.

The $C_{1-6}$alkoxy substituent represents an alkyl group as described above attached through an oxygen bridge.

Examples for the aralkyl and heteroaralkyl substituents recited above are benzyl, phenethyl, 3,3-diphenylpropyl, 3-indolylmethyl.

Halo means chloro, bromo, iodo, or fluoro.

Examples for the heteroaryl substituent are pyridyl, thienyl, furyl, imidazolyl, thiazolyl, indolyl, quinolinyl, isoquinolinyl, benzimidazolyl, benzothiazolyl, and benzthienyl.

Examples for the $C_{1-6}$alkanoylamino and aroylamino substituent are acetylamino and benzoylamino.

The Formula I compounds can be used in the form of salts derived from inorganic or organic acids and bases. Including among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine and N-methyl-D-glucamine, and salts with amino acids such as arginine and lysine. Water or oil-soluble or dispersible products are thereby obtained.

In the compounds of Formula I, the carbon atom to which $R^1$ is attached, as well as the carbon atoms of the $R^3$ substituent, may be asymmetric. These compounds accordingly exist in diastereo-isomeric forms or in mixtures thereof. Although the L- or S-configuration is preferred for each of the asymmetric carbon atoms, diastereomers containing D- or R-amino acids have activity dependent upon their structures and have advantages with respect to metabolic stability and can, therefore, be utilized in mixture or as pure diastereomeric compounds.

Preferred compounds of the present invention are those of Formula I wherein:

$R^1$ and $R^5$ are as defined above;
$R^2$ is amino, $C_{1-6}$alkylamino; di-$C_{1-6}$alkylamino; aryl-$C_{1-6}$alkylamino and arylamino wherein aryl is as defined above, hydroxy, $C_{1-6}$alkoxy, carboxy-$C_{1-6}$-alkylamino, carboxamido-$C_{1-6}$alkylamino;
$R^3$ is methylene or ethylene;
$R^4$ is hydroxy; $C_{1-6}$alkanoyloxy; aryl-$C_{1-6}$alkanoyloxy wherein aryl is as defined above; and,
m is 1, 2, or 3.

Especially preferred compounds are
1-(3-acetoxy-2-oxopropyl)-3-(1(S)-carboethoxy-3-phenyl-1-propyl)-(S)-aminohomodihydrocarbostyril;
1-(3-hydroxy-2-oxopropyl)-3-(1(S)-carboethoxy-3-phenyl-1-propyl)-(S)-aminohomodihydrocarbostyril; and,
1-(3-hydroxy-2-oxopropyl)-3-(1(S)-carboxy-3-phenyl-1-propyl)-(S)-aminohomodihydrocarbostyril.

The compounds of this invention inhibit angiotensin converting enzyme (ACE) and thus block conversion of the decapeptide angiotensin I to angiotensin II. Angiotensin II is a potent pressor substance. Thus blood pressure lowering can result from inhibition of its biosynthesis especially in animals and humans whose hypertension is angiotensin II related. Furthermore, converting enzyme degrades the vasodilator peptide, bradykinin. Therefore, inhibitors of ACE may lower blood pressure also by potentiation of bradykinin. Although the relative importance of these and other possible mechanisms remains to be established, inhibitors of ACE are effective antihypertensive agents in a variety of animal models and are useful clinically, for example, in many human patients with renovascular, malignant and essential hypertension. See, for example, D. W. Cushman et al., Biochemistry 16, 5484 (1977).

The evaluation of converting enzyme inhibitors is guided by in vitro enzyme inhibition assays. For example, a useful method is that of Y. Piquilloud, A. Reinharz and M. Roth, Biochem. Biophys. Acta, 206, 136 (1970) in which the hydrolysis of carbobenzyloxyphenylalanylhistidinylleucine is measured. In vivo evaluations may be made, for example, in normotensive rats challenged with angiotensin I by the technique of J. R. Weeks and J. A. Jones, Proc. Soc. Exp. Biol. Med., 104, 646 (1960) or in a high renin rat model such as that of S. Koletsky et al., Proc. Soc. Exp. Biol. Med., 125, 96 (1967).

Thus, the compounds of the invention are useful in treating hypertension. They are also of value in the

management of acute and chronic congestive heart failure, in the treatment of secondary hyperaldosteronism, primary and secondary pulmonary hypertension, renal failure and renal vascular hypertension, and in the management of vascular disorders such as migraine or Raynaud's disease. The application of the compounds of this invention for these and similar disorders will be apparent to those skilled in the art.

An embodiment of this invention is the use of the compounds of Formula I for the treatment and the prevention of disorders of the gastrointestinal, central nervous, and appetite regulatory systems of mammals, especially of man. Specifically, these Formula I compounds are useful in treatment and prevention of disorders of gastric acid secretion, gastrointestinal motility, pancreatic secretions, and dopaminergic functions. The compounds of Formula I are especially useful in the prevention and treatment of irritable bowel syndrome.

The ability of the compounds of Formula I to antagonize CCK and gastrin makes these compounds especially useful in the treatment and prevention of disease states wherein CCK or gastrin may be involved, for example, gastrointestinal disorders such as irritable bowel syndrome, ulcers, excess pancreatic or gastric secretion, acute pancreatis, motility disorders, central nervous system disorders caused by CCK's interaction with dopamine such as neuroleptic disorders, tardive, dyskinesia, Parkinson's disease, psychosis or Gilles de la Tourette Syndrome, and disorders of appetite regulatory systems.

CCK In Vitro Activity of Formula I Compounds

The CCK biological activity of the compounds of Formula I have been evaluated using an $^{125}$I-CCK receptor binding assay and *in vitro* isolated tissue preparations.

Materials and Methods

1. CCK Receptor Binding (Pancreas)

CCK-33 was radiolabeled with $^{125}$I-Bolton Hunter reagent (2000 Ci/mmole) as described by Sankara et al. (J. Biol. Chem. 254: 9349-9351, 1979). Receptor binding was performed according to Innis and Snyder (Proc. Natl. Acad. Sci. 77: 6917-6921, 1980) with the minor modification of adding the additional protease inhibitors, phenylmethane sulfonyl fluoride and o-phenanthroline. The latter two compounds have no effect on the $^{125}$I-CCK receptor binding assay.

Male Sprague-Dawley rats (200-350g) were sacrificed by decapitation. The whole pancreas was dissected free of fat tissue and was homogenized in 20 volumes of ice-cold 50 mM, Tris HCl (pH 7.7 at 25°C) with a Brinkmann Polytron PT 10. The homogenates were centrifuged at 48,000 g for 10 min. Pellets were resuspended in Tris Buffer, centrifuged as above and resuspended in 200 volumes of binding assay buffer (50 mM Tris HCl, pH 7.7 at 25°C, 5 mM dithiothreitoe, 0.1 mM bacitracin, 1.2 mM phenylmethane sulfonyl fluoride and 0.5 mM o-phenanthroline). For the binding assay, 25 µl of buffer (for total binding) or unlabeled CCK-8 sulfate to give a final concentration of 1 µM (for nonspecific binding) or the compounds of Formula I (for determination inhibition of $^{125}$I-CCK binding) and 25 µl of $^{125}$I-CCK-33 (30,000-40,000 cpm) were added to 450 µl of the membrane suspensions in microfuge tubes. All assays were run in duplicate or triplicate. The reaction mixtures were incubated at 37°C for 30 minutes and centrifuged in a Beckman Microfuge (4 minutes) immediately after adding 1 ml of ice-cold incubation buffer. The supernatant was aspirated and discarded, pellets were counted with a Beckman gamma 5000.

2. CCK Receptor Binding (Brain)

CCK-33 was radiolabeled and the binding was performed according to the description for the pancreas method with modifications according to the Saito, et al. (J. Neurochem, 37, 483-490 (1981)).

Male Hartley guinea pigs (300-500g) were sacrificed by decapitation and the brains were removed and placed in ice-cold 50 mM, Tris HCl plus 7.58 g/L Trizma-7.4 (pH 7.4 at 25°C) Cerebral cortex was dissected and used as a receptor source. Each gram of fresh guinea pig brain tissue was homogenized in 10 ml of Tris/Trizma buffer with a Brinkman polytron PT-10. The homogenates were centrifuged at 42,000 g for 15 min. Pellets were resuspended in Tris Buffer, centrifuged as above and resuspended in 200 volumes of binding assay buffer (10 mM HEPES, pH 7.7 at 25°C, 5 mM $MgCl_2$, 1 mM EGTA 0.4% BSA (bovine serum albumin) , and 0.25 mg/ml bacitracin). For the binding assay, 25 µl of buffer (for total binding) or unlabeled CCK-8 sulfate to give a final concentration of 1µM (for nonspecific binding) or the compounds of Formula I (for determination inhibition of $^{125}$I-CCK binding) and 25 µl of $^{125}$I-CCK-33 (30,000-40,000 cpm) were added to 450 µl of the membrane suspensions in microfuge tubes. All assays were run in duplicate or

triplicate. The reaction mixtures were incubated at 25°C for 2 hours and centrifuged in a Beckman Microfuge (4 minutes) immediately after adding 1 ml of ice-cold incubation buffer. The supernatant was aspirated and discarded, pellets were counted with a Beckman gamma 5000.

In Vitro Results

1. Effect of The Compounds of Formula I on $^{125}$I-CCK-33 receptor binding

Compounds of Formula I inhibited specific $^{125}$I-CCK-33 binding in a concentration dependent manner with an $IC_{50}$ less than or equal to 100 $\mu$M such as, for example:
1-(3-acetoxy-2-oxopropyl)-3-(1-carboethoxy-3-phenyl-1-propyl)aminohomodihydrocarbostyril, $IC_{50}$ = 25 $\mu$M.

Thus, in accordance with the present invention there is provided a pharmaceutical composition for treating hypertension or as cholecystokinin antagonists comprising a pharmaceutically acceptable carrier and a pharmaceutically effective amount of a compound of Formula I.

There is also provided, in accordance with the present invention, a method of treating hypertension or a method of treating gastrointestinal disorders, central nervous system disorders, or regulating appetite which comprises administering to a patient in need of such treatment a pharmaceutically effective amount of a compound of Formula I.

The compound is preferably a member of the group 1-(3-acetoxy-2-oxopropyl)-3-(1-carboethoxy-3-phenyl-1-propyl)-aminohomodihydrocarbostyril;
1-(3-hydroxy-2-oxopropyl)-3-(1-carboethoxy-3-phenyl-1-propyl)-aminohomodihydrocarbostyril;
1-(3-acetoxy-2-oxopropyl)-3-[1-carboethoxy-3-(3-indolyl)-1-propyl]aminohomodihydrocarbostyril;
1-(3-acetoxy-2-oxopropyl)-3-[1-N(n)hexylcarboxamido-3-(3-indolyl)-1-propyl]aminohomodihydrocarbostyril;
and
1-(3-hydroxy-2-oxopropyl)-3-[1-N(n)hexylcarboxamido-3-(3-indolyl)-1-propyl]aminohomodihydrocarbostyril.

For administration, the compositions of the invention can also contain other conventional pharmaceutically acceptable compounding ingredients, as necessary or desired. Such ingredients are generally referred to as carriers or diluents. Conventional procedures for preparing such compositions in appropriate dosage forms can be utilized. Whatever the dosage form, it will contain a pharmaceutically effective amount of the compounds of the invention.

The present compositions can be administered orally or other than orally; e.g., parenterally, by insufflation, topically or rectally; using appropriate dosage forms; e.g., tablets, capsules, suspensions or solutions for oral administration; suspension and emulsion for parenteral administration; solutions for intravenous administration; and ointments or transdermal patches for topical administration.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets containing the active ingredient in admixture with non-toxic pharmaceutically acceptable excipients may also be manufactured by known methods. The excipients used may be for example, (1) inert diluents such as calcium carbonate, sodium carbonate, lactose, calcium phosphate or sodium phosphate; (2) granulating and disintegrating agents such as corn starch, or alginic acid; (3) binding agents such as starch, gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patents 4,256,108; 4,160,452; and 4,265,874 to form osmotic therapeutic tablets for controlled release.

In some cases, formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, for example peanut oil, liquid paraffin, or olive oil.

Aqueous suspensions normally contain the active materials in admixture with excipients suitable for the manufacture of aqueous suspensions. Such excipients may be

(1) suspending agents such as sodium carboxymethylcellulose, methylcellulose, hydroxypropylmethylcellulose, sodium alginate, polyvinylpyrrolidone, gum tragacanth and gum acacia;

(2) dispersing or wetting agents which may be

(a) a naturally-occurring phosphatide such as lecithin,

(b) a condensation product of an alkylene oxide with a fatty acid, for example, polyoxyethylene stearate,

(c) a condensation product of ethylene oxide with a long chain aliphatic alcohol, for example, heptadecaethyleneoxycetanol,

(d) a condensation product of ethylene oxide with a partial ester derived from a fatty acid and a hexitol such as polyoxyethylene sorbitol monooleate, or

(e) a condensation product of ethylene oxide with a partial ester derived from a fatty acid and a hexitol anhydride, for example polyoxyethylene sorbitan monooleate.

The aqueous suspensions may also contain one or more preservatives, for example, ethyl or n-propyl p-hydroxybenzoate; one or more coloring agents; one or more flavoring agents; and one or more sweetening agents such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide a palatable oral preparation. These compositions may be preserved by the addition of an antioxidant such as ascorbic acid.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example, those sweetening, flavoring and coloring agents described above may also be present.

The pharmaceutical compositions of the invention may also be in the form of oil-in-water emulsions. The oily phase may be a vegetable oil such as olive oil or arachis oil, or a mineral oil such as liquid paraffin or a mixture thereof. Suitable emulsifying agents may be (1) naturally-occurring gums such as gum acacia and gum tragacanth, (2) naturally-occurring phosphatides such as soy bean lecithin, (3)esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain a demulcent, a preservative and flavoring and coloring agents.

The pharmaceutical compositions may be in the form of a sterile injectable aqueous or oleaginous suspension. This suspension may be formulated according to known methods using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

A composition of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions containing the compositions of the invention are employed.

Treatment dosage for human beings can be varied as necessary. Generally, daily dosages of the compounds of the invention can range from about 0.5 mg to about 1000 mg; preferably, from about 5 mg to about 500 mg, whether administered as an ACE inhibitor or as a CCK antagonist.

The amount of active ingredient that may be combined with the carrier materials to produce a single dosage form will vary depending upon the host treated and the particular mode of administration. For example, a formulation intended for oral administration may contain from 5 mg to 500 mg of active agent compounded with an appropriate and convenient amount of carrier material which may vary from about 5 to about 95 percent of the total composition.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general

health, sex, diet, time of administration, route of administration, rate of excretion, drug combination and the severity of the particular disease undergoing therapy.

The compounds of this invention when used to treat hypertension can also be administered in combination with other antihypertensives and/or diuretics and/or calcium entry blockers. For example, the compounds of this invention can be given in combination with such compounds as acetazolamide, amiloride, aminophylline, atenolol, bendroflumethiazide, benzthiazide, bumetanide, chlorothalidone, chlorothiazide, clonidine, cryptenamine acetates and cryptenamine tannates, cyclothiazide, deserpidine, diazoxide, diltiazem, (S)-1-[[2-(3,4-dimethoxyphenyl)ethyl]amino]-3-[[4-(2-thienyl)-1H-imidazol-2-yl]-phenoxy]-2-propanol, ethacrynic acid, flumethiazide, furosemide, guanethidene sulfate, hydralazine hydrochloride, hydrochlorothiazide, hydroflumethiazide, (+)-4-[3-[-[2-(1-hydroxycyclohexyl)ethyl]-4-oxo-2-thiazolidinyl]propyl]-benzoic acid, indacrinone and variable ratios of its enantiomers, merethoxylline procaine, methylclothiazide, methyldopa, methyldopate hydrochloride, metolazone, metoprolol tartate, minoxidil, naldolol, nifedipine, pargyline hydrochloride, pindolol, polythiazide, prazosin, propranolol, quinethazone, rauwolfia serpentina, rescinnamine, reserpine, sodium ethacrynate, sodium nitroprusside, spironolactone, ticrynafen, timolol, triamterene, trichlormethiazide, trimethophan camsylate, bepridil, diltiazim, etafenone, falipamil, felodipine, flunarizine, gallopamil, indapamide, lidoflazine, nicardipine, nifedipine, nimopidine, nitrendipine, perhexiline, prenylamine, tiapamil, verapamil, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the antihypertensives of this invention effective in the 5 to 500 mg per day range can be effectively combined with the following compounds at the indicated per day dose range: hydrochlorothiazide (10-100 mg); chlorothiazide (125-2000 mg); manipulated indacrinone enantiomer ratio (25-150 mg); ethacrynic acid (15-2000 mg); amiloride (5-20 mg); furosemide (5-80 mg); propranolol (20-480 mg); timolol (5-60 mg); and methyldopa (65-2000 mg); and the pivaloyloxyethyl ester of methyldopa (30-1000 mg). In addition, triple drug combinations of hydrochlorothiazide (10-100 mg) plus amiloride (5-20 mg) plus converting enzyme inhibitor of this invention (5 - 500 mg); hydrochlorothiazide (10-100 mg) plus timolol (5-60 mg) plus the converting enzyme inhibitor of this invention (5 - 500 mg); or manipulated indacrinone enantiomer ratio (25-150 mg) plus amiloride (5-20 mg) plus converting enzyme inhibitor of this invention (5 - 500 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

The compounds of Formula I can be prepared by the method shown in the following Reaction Scheme wherein $R^1$-$R^5$ and m are as defined above unless otherwise indicated.

As will be evident to those skilled in the art and as demonstrated in the Examples hereinafter, reactive groups not involved in the reactions, such as amino, carboxy or mercapto., may be protected by methods standard in peptide chemistry prior to the coupling reactions and subsequently deprotected to obtain the desired products.

REACTION SCHEME

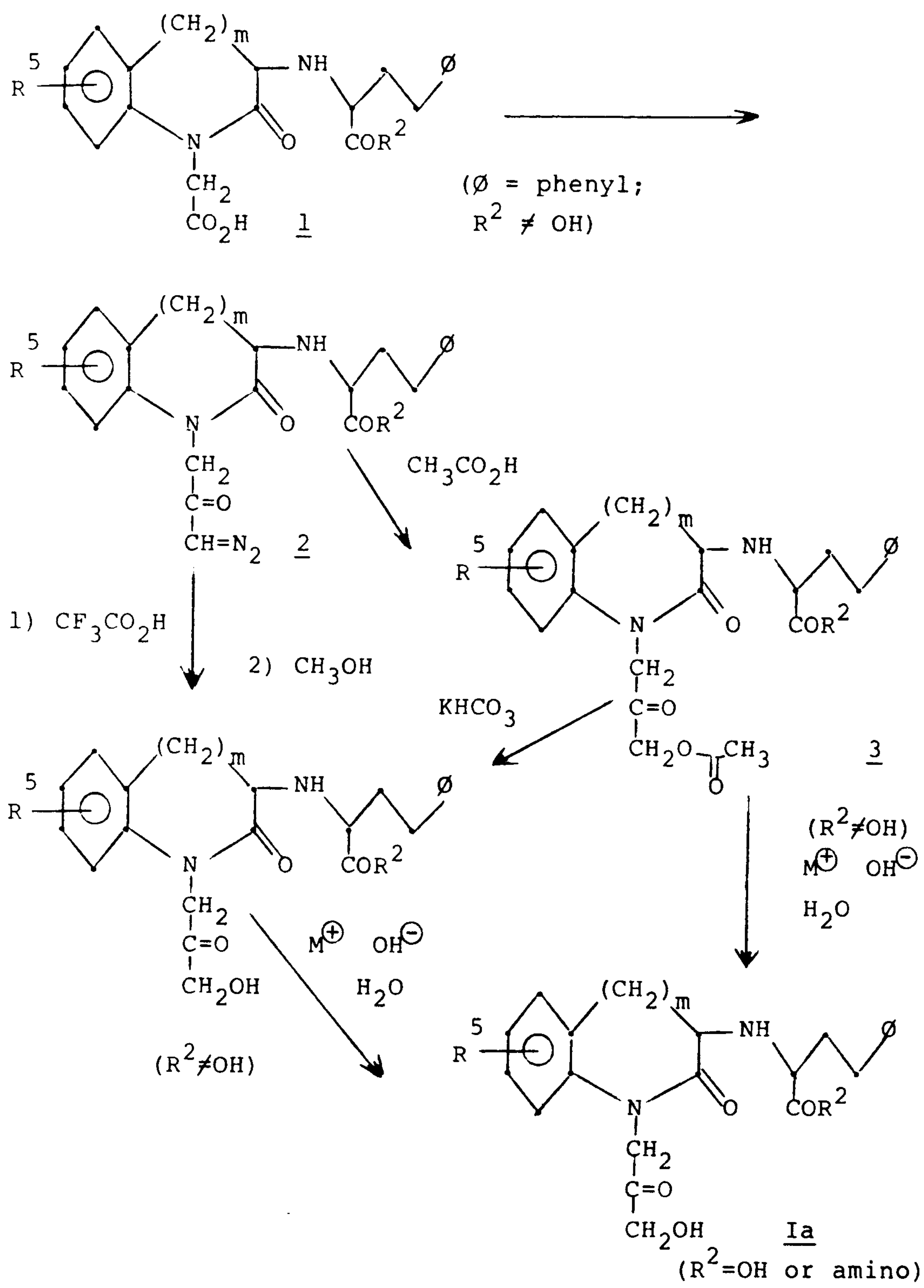


As shown in the above Reaction Scheme, benzolactam mono acid 1 [prepared according to the procedures disclosed in U.S. Patent 4,410,520] can be reacted with an alkoxy chloro formate (such as ethyl or methyl chloro formate) and a tertiary amine (such as triethylamine) in an aprotic solvent (such as tetrahydrofuran (THF)) to give, upon filtration, a mixed anhydride intermediate which is then reacted with an excess of diazomethane in ether to give diazomethylketone 2. Reaction of 2 in boiling acetic acid produces

acetate 3 which, upon treatment with a metal hydroxide (such as sodium or lithium hydroxide), in a protic solvent (such as methanol and water) affords hydroxy methylketone acid Ia.

Alternatively, diazomethylketone 2 can be stirred in trifluoroacetic acid (TFA) followed by stirring in methanol or ethanol to give hydroxymethylketone ester 4.

Reaction of 4 with sodium hydroxide and water in a protic solvent (such as ethanol) provides a compound Ia of the invention.

Hydroxyketone ester 4 can also be prepared from acetate 3 by treating 3 with potassium or sodium bicarbonate in water.

In the above preparation, the keto acid or ester can be represented by the formula

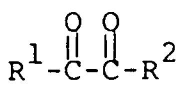

and may be, for example, 2-oxo-4-phenyl-butyric acid. Other α-keto acids or esters may be utilized to prepare other compounds of the present invention for various definitions of $R^1$ and $R^2$. Such α-keto acids are readily available or may be prepared by well-known techniques. For example, synthons such as

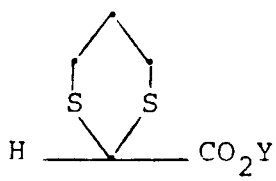

can be converted to α-keto acids or esters using methods involving alkylation followed by hydrolysis as described in the literature. An excellent method involves the reaction of Grignard reagents $R_1MgX$ with $ClCOCO_2Y$ or $YO_2CCO_2Y$. Another method involves condensing substituted acetic acid esters with diethyl oxalate followed by hydrolytic decarboxylation under acidic conditions to obtain α-keto acids. Carefully controlled acid hydrolysis in alcohol of acyl cyanides, which are prepared from acid chlorides and cuprous cyanide, also proves to be a viable synthetic route to α-keto esters. Nucleophilic displacement reactions on chloro or bromo pyruvic acid (ester) can also be used to produce a variety of interesting α-keto acids (esters) . In these formulae, Y is a group such as loweralkyl or benzyl and protecting groups are employed as necessary in the $R_1$ group if an interfering functionality is present.

Additional compounds of Formula I can be prepared by employing the keto acids and esters listed in Table I below.

TABLE I

Keto Acids and Esters of the Formula $R^1$-C=O / COOR (II.)

(a)

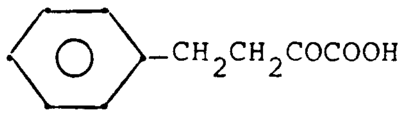

(b)

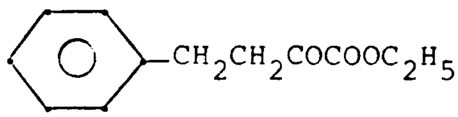

(c)

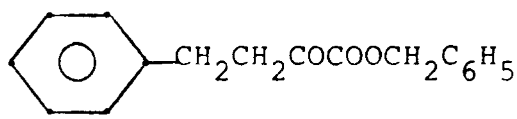

(d)

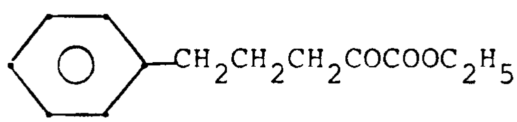

(e)

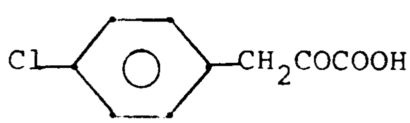

(f)

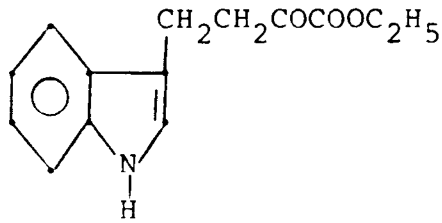

(g)

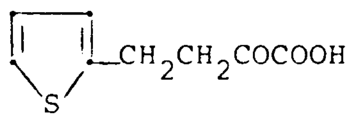

(h)

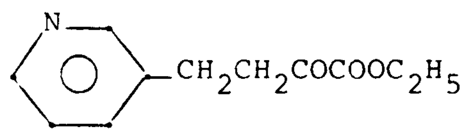

(i)

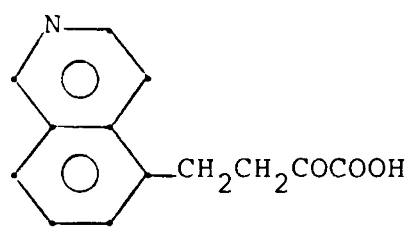

(j)

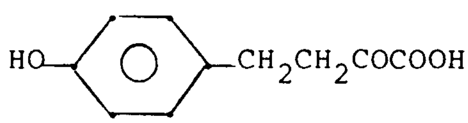

(k)

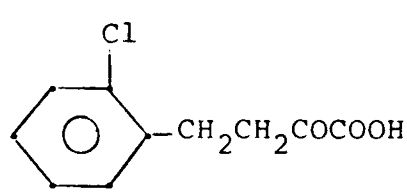

(l)

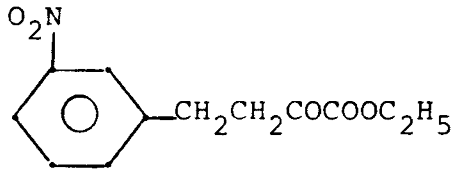

(precursor for the corresponding amino compound)

(m)

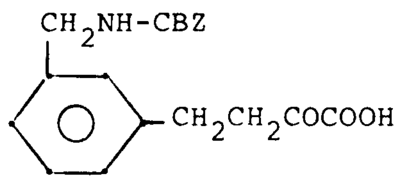

(precursor for the corresponding amino compound)

(n)

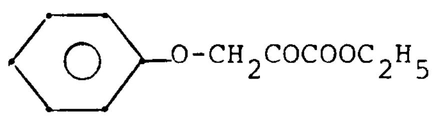

(o)

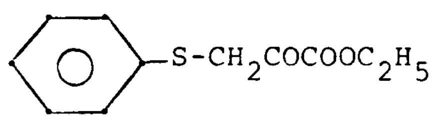

(p)

$CH_3S-CH_2CH_2COCOOH$

(q)

$(CH_3)_2-CH_2CH_2COCOOH$

(r)

$CBZ-HN(CH_2)_4-COCOOH$

(precursor for the corresponding amino compound)

(s)

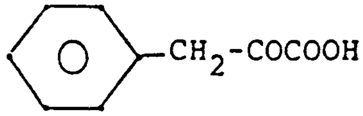

(t)

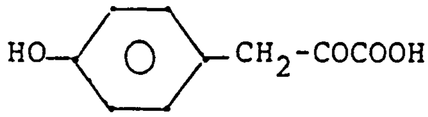

(u)

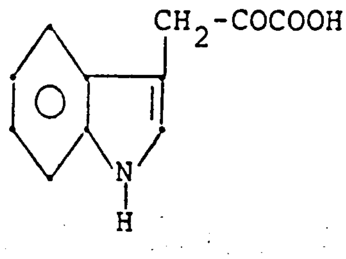

The following examples set forth the best mode currently known for preparing the compounds of the invention. Unless otherwise indicated, all temperatures are in degrees Celsius.

EXAMPLE 1

1-(3-Diazo-2-oxopropyl)-3-(1-carboethoxy-3-phenylpropyl)amino homodihydrocarbostyril

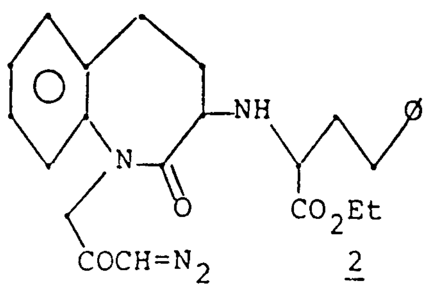

2

To a solution of 0.5 gm of 1-carboxymethyl-3-(1-carboethoxy-3-phenyl-propyl)amino homodihydrocarbostyril (racemate no. 2) in 5 mL of THF at 0 ° C there was added with stirring 0.167 mL of triethylamine followed by 0.097 mL of methyl chloro formate. Stirring at 0 ° C was continued for 15 minutes whereupon the reaction mixture was filtered in a nitrogen atmosphere to remove the precipitated triethylamine hydrochloride. To the remaining solution at 0 ° C there was then added a solution of freshly prepared diazomethane in ether. The solution was then stirred 12 hours at 0 ° C. The reaction's progress was monitored by TLC (silica, 1:1 hexanes: ethylacetate) and diazomethane solution was added as needed. When the reaction was complete, the solution was filtered and she solvents removed at reduced pressure. The crude reaction product was purified by chromatography, (silica, 1:1, hexanes:ethylacetate) to give 0.470 gm of diazoketone 1. 87% TLC (2:1, ethylacetate:hexanes) $R_f = 0.50$, NMR ($CDCl_3$, TMS) 1.21 (t, 3H); 1.8-2.8 (m, 8H); 3.0-3.4 (m, 3H); 4.1 (q, 2H); 4.42 (q, 2H); 5.58 (s, 1H); 7.06 (2s, 9H).

EXAMPLE 2

1-(3-Acetoxy-2-oxopropyl)-3-(1-carboethoxy-3-phenyl-1-propyl)amino homodihydrocarbostyril

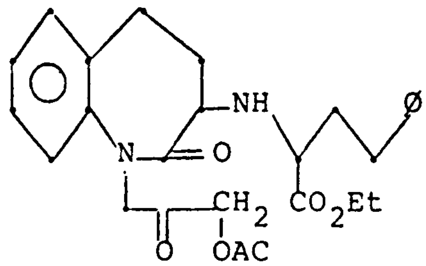

A solution of 0.3 gms of the diazoketone (2) from Example 1 in 5 mL of Acetic acid was refluxed for 1 hour at which time the acetic acid was removed *in vacuo*. The crude product was chromatographed (silica, 3:1, ethylacetate:hexanes) to give 0.25 gm of the title compound.
TLC (silica, ethylacetate) $R_f = 0.73$

| An. Calc. for $C_{27}H_{32}N_2O_6$ | | | |
|---|---|---|---|
| | N, 5.83; | C, 67.48; | H, 6.71. |
| Found: | N, 5.60; | C, 67.19; | H, 6.73. |

NMR ($CDCl_3$, TMS) 1.2 (t, 3H), 1.7-3.0 (m, 7H); 2.2 (s, 3H); 3.0-3.6 (m, 3H); 4.0 (q, 2H); 3.8-4.2 (m, 1H); 4.6 (s, 2H); 4.8 (s, 2H); 7.2 (s, 9H).

EXAMPLE 3

1-(3-Hydroxy-2-oxopropyl)-3-(1-carboethoxy-3-phenyl-1-propyl)amino homodihydrocarbostyril

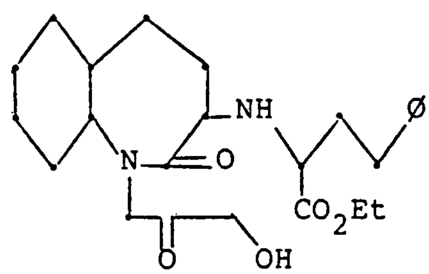

A solution of 1.2 gm of the diazoketone 2 from Example 1 in 10 mL of trifluoroacetic acid was stirred at room temperature for 3 hours at which time the solvent was removed at reduced pressure. The crude reaction mixture was dissolved in 10 mL of methanol and the solution stirred 6 hours at room temperature whereupon the methanol was removed *in vacuo*. The crude product was chromatographed (silica, ethylacetate) to give 0.7 gm of the title of product.
TLC (silica, ethylacetate ) $R_f = 0.58$.
NMR ($CDCl_3$, TMS) 1.1 (t, 3H); 1.6-3.0 (m, 6H); 3.0-3.15 (m, 3H); 4.0 (q,2H); 4.0-4.3 (m, 1H); 4.3 (s, 2H); 4.6 (s, 2H); 7.1 (s, 9H).

EXAMPLE 4

1-(3-Hydroxy-2-oxopropyl)-3-(1-carboxy-3-phenyl-1-propyl)amino homodihydrocarbostyril

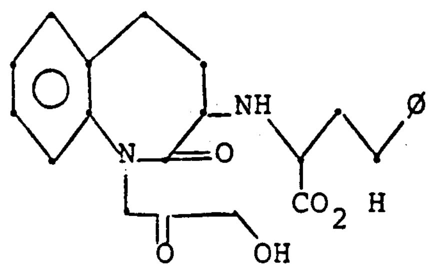

To a solution of 0.075 gm of the compound from Example 3 in 0.3 mL of methanol and 0.266 mL of a 3N solution of sodium hydroxide was stirred for 12 hours at room temperature. The $H_2O$ and methanol was evaporated at reduced pressure. The crude product was dissolved in 1 mL of $H_2O$ and carefully acidified with acetic acid. A white solid precipitated upon trituration, and, upon filtration and drying in vacuo, 40 mg (59%) of the titled product was isolated.
TLC (silica, 85:85:15 ethanol:methylene chloride:ammonium hydroxide) $R_f = 0.45$.

| An. Calc. for $C_{23}H_{26}N_2O_5 \cdot$ 1-3/4 $H_2O$ | | | |
|---|---|---|---|
| | N, 6.34; | C, 62.52; | H, 5.93. |
| Found: | N, 6.36; | C, 62.56; | H, 6.05. |

NMR ($D_2O$, NaOD) 1.6-3.0 (m, 6H); 3.0-3.5 (m, 3H); 3.9-4.5 (m, 5H); 7.1 (bs, 9H).

**Claims**

**1.** A compound of the formula:

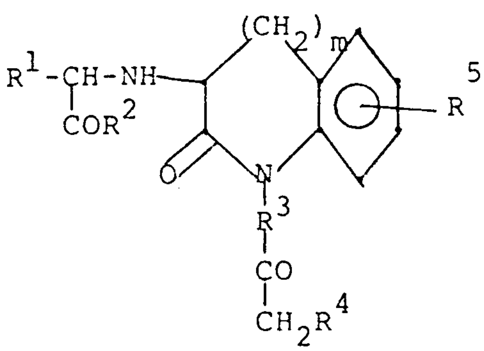

I

wherein:

$R^1$ is hydrogen;
hydrocarbon of from 1 to 12 carbon atoms which include branched and unsaturated alkyl groups;
$C_3$-$C_{10}$-cycloalkyl;
substituted $C_{1-6}$ alkyl wherein the substituent can be halo, hydroxy, carboxy, $C_{1-6}$alkylthio, $C_{1-6}$alkoxy, $C_{1-6}$alkoxycarbonyl, carboxamido, $C_{1-6}$aralkoxycarbonyl wherein aryl is phenyl, naphthyl or biphenyl, amino, $C_{1-6}$alkylamino, $C_{1-6}$dialkylamino, or $C_{1-6}$alkanoylamino and aroylamino wherein aryl in phenyl, naphthyl or biphenyl;
substituted loweralkyl having the formula $R_A^1$ $(CH_2)_p$-Q-$(CH_2)_q$ wherein p is 0-2, q is 1-3, $R_A^1$ is aryl or heteroaryl wherein aryl is phenyl, naphthyl or biphenyl and heteroaryl is a 5- or 6-membered aromatic ring containing from 1 to 3 heteroatoms selected from the group consisting of nitrogen, oxygen and sulfur and bicyclic groups in which the above-mentioned 5- or 6-membered aromatic rings are fused to a benzene ring and which aryl or heteroaryl

group is optionally substituted by amino, $C_{1-6}$dialkylamino,$C_{1-6}$alkylamino, hydroxy, hydroxy $C_{1-6}$alkyl, amino $C_{1-6}$alkyl, trihalo $C_{1-6}$alkyl, cyano, nitro, sulfonamido, aroyl wherein aryl is as defined above, $C_{1-6}$alkyl, halo, dihalo, and $C_{1-6}$alkoxy, and Q is O, S, N-$R_B^1$, CON$R_C^1$, N$R_C^1$ CO, CH=CH

wherein $R_B^1$ is hydrogen, $C_{1-6}$alkyl, aryl as defined above, aralkyl wherein the alkyl portion is $C_{1-6}$alkyl and the aryl portion is as defined above, $C_{1-6}$alkanoyl, or aroyl wherein aryl is as defined above, and $R_C^1$ is hydrogen, or $C_{1-6}$alkyl;

aryl as defined above;

substituted aryl as defined above wherein the substituent is $C_{1-6}$alkyl, amino $C_{1-6}$alkyl, $C_{1-6}$alkoxy, aryloxy wherein aryl is as defined above, aroyl wherein aryl is as defined above hydroxy, halo, or dihalo;

substituted or unsubstituted aralkyl or substituted or unsubstituted heteroaralkyl wherein the alkyl portion in $C_{1-6}$alkyl and the aryl portion and heteroaryl portion are as defined above and which include branched $C_{1-6}$alkyl groups wherein the $C_{1-6}$alkyl groups can be substituted by amino, $C_{1-6}$alkanoylamino and aroylamino wherein aryl is as defined above, or hydroxyl and the aryl and heteroaryl groups can be substituted by halo, dihalo, $C_{1-6}$alkyl, hydroxy, $C_{1-6}$alkoxy, aryloxy wherein aryl is as defined above, aroyl wherein aryl is as defined above, arylthio wherein aryl is as defined above, amino, amino $C_{1-6}$alkyl, $C_{1-6}$alkanoylamino, aroylamino wherein aryl is as defined above, $C_{1-6}$dialkylamino, $C_{1-6}$alkylamino, hydroxy $C_{1-6}$alkyl, trihalo $C_{1-6}$alkyl, nitro, cyano, or sulfonamido;

$R^2$ is hydroxy; $C_{1-6}$alkoxy;

$C_{2-6}$alkenyloxy; aryl $C_{1-6}$alkoxy wherein aryl is as defined above;

$C_{1-6}$alkanoyl-$C_{1-6}$alkoxy; amino; $C_{1-6}$alkylamino; di $C_{1-6}$alkylamino; aryl-$C_{1-6}$alkylamino wherein aryl is as defined as above; arylamino wherein aryl is as defined above; carboxy $C_{1-6}$alkylamino; carboxamido $C_{1-6}$alkylamino; or aryloxy, or mono- or disubstituted aryloxy wherein aryl is as defined above and wherein the substituents are halo;

m is 1-3;

$R^3$ is $C_{1-6}$alkyl or substituted $C_{1-6}$alkyl wherein the substituents are hydrogen, $C_{1-6}$alkyl, or aryl as defined above;

$R^4$ is hydroxy; $C_{1-6}$alkanoyloxy; aryl-$C_{1-6}$alkanoyloxy, wherein aryl is as defined above;

$R^5$ is hydrogen; halo; hydroxy; $C_{1-6}$alkyl; or $C_{1-6}$alkoxy; and

a pharmaceutically acceptable salt thereof.

**2.** A compound of Claim 1 which is a member of the group:
1-(3-acetoxy-2-oxopropyl)-3-(1(S)-carboethoxy-3-phenyl-1-propyl)-(S)-aminohomodihydrocarbostyril;
1-(3-hydroxy-2-oxopropyl)-3-(1(S)-carboethoxy-3-phenyl-1-propyl)-(S)-aminohomodihydrocarbostyril;
and,
1-(3-hydroxy-2-oxopropyl)-3-(1(S)-carboxy-3-phenyl-1-propyl)-(S)-aminohomodihydrocarbostyril.

**3.** A pharmaceutical composition useful in treating hypertension comprising a pharmaceutically acceptable carrier and an antihypertensively effective amount of a compound of Claim 1.

**4.** A composition according to Claim 3 which includes an antihypertensive and/or diuretic and/or calcium entry blocker compound selected from the group consisting of acetazolamide, amiloride, aminophylline, atenolol, bendroflumethiazide, benzthiazide, bumetanide, chlorothalidone, chlorothiazide, clonidine, cryptenamine acetates and cryptenamine tannates, cyclothiazide, deserpidine, diazoxide, diltiazem, (S)-1-[[2-(3,4-dimethoxyphenyl)-ethyl]-amino]-3-[[4-(2-thienyl)-1H-imidazol-2-yl]-phenoxy]-2-propanol, ethacrynic acid, flumethiazide, furosemide, guanethidene sulfate, hydralazine hydrochloride, hydrochlorothiazide, hydroflumethiazide, (+)-4-[3-[-[2-(1-hydroxycyclohexyl)-ethyl]4-oxo-2-thiazolidinyl]propyl]-benzoic acid; indacrinone and variable ratios of its enantiomers, merethoxylline procaine, methylclothiazide, methyldopa, methyldopate hydrochloride, metolazone, metoprolol tartate, minoxidil, naldolol, nifedipine, pargyline hydrochloride, pindolol, polythiazide, prazosin, propranolol, quinethazone, rauwolfia serpentina, rescinnamine, reserpine, sodium ethacrynate, sodium nitroprusside, spironolactone, ticrynafen, timolol, triamterene, trichlormethiazide, trimethophan camsylate, bepridil, diltiazim, etafenone, falipamil, felodipine, flunarizine, gallopamil, indapamide, lidoflazine, nicardipine, nifedipine, nimopidine, nitrendipine, perhexiline, prenylamine, tiapamil, verapamil, as well as admixtures and

combinations thereof.

**5.** A pharmaceutical composition useful for treating gastrointestinal disorders, central nervous system disorders, or regulating appetite in humans, comprising a pharmaceutically effective amount of a compound of Claim 1 and an acceptable pharmaceutical carrier.

**6.** The composition of Claim 5 wherein said compound is a member of the group:
1-(3-acetoxy-2-oxopropyl)-3-(1-carboethoxy-3-phenyl-1-propyl)aminohomodihydrocarbostyril;
1-(3-hydroxy-2-oxopropyl)-3-(1-carboethoxy-3-phenyl-1-propyl)aminohomodihydrocarbostyril;
1-(3-acetoxy-2-oxopropyl)-3-[1-carboethoxy-3-(3-indolyl)-1-propyl]aminohomodihydrocarbostyril;
1-(3-acetoxy-2-oxopropyl)-3-[1-N(n)hexylcarboxamido-3-(3-indolyl)-1-propyl]-aminohomodihydrocarbostyril; and,
1-(3-hydroxy-2-oxopropyl)-3-[1-N(n)hexylcarboxamido-3-(3-indolyl)-1-propyl]-aminohomodihydrocarbostyril.

## Revendications

**1.** Composé de formule :

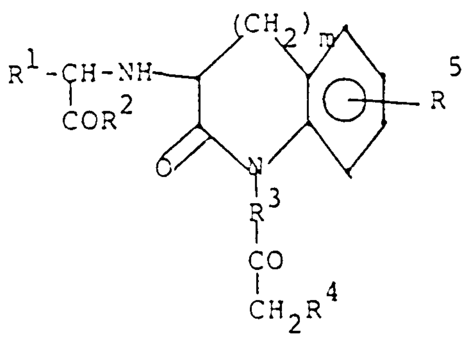

I

dans laquelle :

$R^1$ est un hydrogène ;
un hydrocarbure de 1 à 12 atomes de carbone, y compris les groupes alkyles ramifiés et insaturés ;
un cycloalkyle en $C_3$-$C_{10}$ ;
un alkyle en $C_1$-$C_6$ substitue dont le substituant peut être halogéno, hydroxy, carboxy, alkylthio en $C_1$-$C_6$, alcoxy en $C_1$-$C_6$, (alcoxy en $C_1$-$C_6$)carbonyle, carboxamido, (aralcoxy en $C_1$-$C_6$)carbonyle dont l'aryle est phényle, naphtyle ou biphényle, amino, alkylamino en $C_1$-$C_6$, di(alkyl en $C_1$-$C_6$)amino ou alcanoylamino en $C_1$-$C_6$ et aroylamino dont l'aryle est phényle, naphtyle ou biphényle ;
un alkyle inférieur substitué de formule $R_A^1$ $(CH_2)_p$-Q-$(CH_2)_q$, où p est 0-2, q est 1-3, $R_A^1$ est un aryle ou un hétéroaryle, l'aryle étant un phényle, naphtyle ou biphényle et l'hétéroaryle étant un cycle aromatique à 5 ou 6 chaînons contenant de 1 à 3 hétéroatomes choisis parmi l'azote, l'oxygène et le soufre, et les groupes bicycliques dans lesquels les cycles aromatiques à 5 ou 6 chaînons précités sont condensés à un cycle benzène et dont le groupe aryle ou hétéroaryle est facultativement substitué par amino, di(alkyl en $C_1$-$C_6$)amino, alkylamino en $C_1$-$C_6$, hydroxy, hydroxyalkyle en $C_1$-$C_6$, aminoalkyle en $C_1$-$C_6$, trihalogénoalkyle en $C_1$-$C_6$, cyano, nitro, sulfonamido, aroyle dont l'aryle est comme défini ci-dessus, alkyle en $C_1$-$C_6$, halogéno, dihalogéno et alcoxy en $C_1$-$C_6$, et Q est O, S, N-$R_B^1$, CON$R_C^1$, N$R_C^1$ CO, CH=CH, où $R_B^1$ est un hydrogène, un alkyle en $C_1$-$C_6$, un aryle comme défini ci-dessus, un aralkyle dont la portion alkyle est un alkyle en $C_1$-$C_6$ et la portion aryle est comme défini ci-dessus, un alcanoyle en $C_1$-$C_6$ ou un aroyle dont l'aryle est comme défini ci-dessus et $R_C^1$ est un hydrogène ou un alkyle en $C_1$-$C_6$ ;
un aryle comme défini ci-dessus ;
un aryle substitué comme défini ci-dessus dont le substituant est un alkyle en $C_1$-$C_6$, un

aminoalkyle en $C_1$-$C_6$, un alcoxy en $C_1$-$C_6$, un aryloxy dont l'aryle est comme défini ci-dessus, un aroyle dont l'aryle est comme défini ci-dessus, un hydroxy, un halogéno ou un dihalogéno ; un aralkyle substitué ou non substitué ou un hétéroaralkyle substitué ou non substitué dont la portion alkyle est un alkyle en $C_1$-$C_6$ et la portion aryle est comme défini ci-dessus et qui comprennent les groupes alkyles en $C_1$-$C_6$ ramifiés où les groupes alkyles en $C_1$-$C_6$ peuvent être substitués par amino, alcanoylamino en $C_1$-$C_6$ et aroylamino dont l'aryle est comme défini ci-dessus, ou hydroxyle et les groupes aryles et hétéroaryles peuvent être substitués par halogéno, dihalogéno, alkyle en $C_1$-$C_6$ hydroxy, alcoxy en $C_1$-$C_6$, aryloxy dont l'aryle est comme défini ci-dessus, aroyle dont l'aryle est comme défini ci-dessus, arylthio dont l'aryle est comme défini ci-dessus, amino, aminoalkyle en $C_1$-$C_6$, alcanoylamino en $C_1$-$C_6$, aroylamino dont l'aryle est comme défini ci-dessus, di(alkyl en $C_1$-$C_6$)amino, alkylamino en $C_1$-$C_6$, hydroxyalkyle en $C_1$-$C_6$, trihalogénoalkyle en $C_1$-$C_6$, nitro, cyano ou sulfonamido ;

$R^2$ est un hydroxy ; un alcoxy en $C_1$-$C_6$ ; un alcényloxy en $C_2$-$C_6$ ; un aryl-alcoxy en $C_1$-$C_6$ dont l'aryle est comme défini ci-dessus ; un (alcanoyl en $C_1$-$C_6$)alcoxy en $C_1$-$C_6$ ; un amino ; un alkylamino en $C_1$-$C_6$ ; un di(alkyl en $C_1$-$C_6$)amino ; un aryl-alkylamino en $C_1$-$C_6$ dont l'aryle est comme défini ci-dessus ; un arylamino dont l'aryle est comme défini ci-dessus ; un carboxy-alkylamino en $C_1$-$C_6$ ; un carboxamidoalkylamino en $C_1$-$C_6$ ; ou un aryloxy ou un aryloxy mono- ou disubstitué dont l'aryle est comme défini ci-dessus et où les substituants sont halogéno ;

m est 1 à 3 ;

$R^3$ est un alkyle en $C_1$-$C_6$ ou un alkyle en $C_1$-$C_6$ substitué dont les substituants sont un hydrogène, un alkyle en $C_1$-$C_6$ ou un aryle comme défini ci-dessus ;

$R^4$ est un hydroxy ; un alcanoyloxy en $C_1$-$C_6$ ; un aryl-alcanoyloxy en $C_1$-$C_6$ dont l'aryle est comme défini ci-dessus ;

$R^5$ est un hydrogène ; un halogéno ; un hydroxy ; un alkyle en $C_1$-$C_6$ ; ou un alcoxy en $C_1$-$C_6$ ; et

un de ses sels pharmaceutiquement acceptables.

**2.** Composé selon la revendication 1 qui fait partie du groupe constitué par :
le 1-(3-acétoxy-2-oxopropyl)-3-(1(S)-carboéthoxy-3-phényl-1-propyl)-(S)-aminohomodihydrocarbostyryle ;
le 1-(3-hydroxy-2-oxopropyl)-3-(1(S)-carboéthoxy-3-phényl-1-propyl)-(S)-aminohomodihydrocarbostyryle ; et
le 1-(3-hydroxy-2-oxopropyl)-3-(1(S)-carboxy-3-phényl-1-propyl)-(S)-aminohomodihydrocarbostyryle.

**3.** Composition pharmaceutique utile dans le traitement de l'hypertension comprenant un véhicule pharmaceutiquement acceptable et une quantité antihypertensive efficace d'un composé selon la revendication 1.

**4.** Composition selon la revendication 3 qui comprend un composé antihypertensif et/ou diurétique et/ou bloquant l'entrée du calcium, choisi dans le groupe constitué par : l'acétazolamide, l'amiloride, l'aminophylline, l'aténolol, le bendrofluméthiazide, le benzthiazide, le bumétanide, la chlorothalidone, le chlorothiazide, la clonidine, les acétates de crypténamine et les tannates de crypténamine, le cyclothiazide, la déserpidine, le diazoxide, le diltiazem, le (S)-1-[[2-(3,4-diméthoxyphényl)éthyl]amino]-3-[[4-(2-thiényl)-1H-imidazole-2-yl]phénoxy]-2-propanol, l'acide éthacrynique, le fluméthiazide, le furosémide, le sulfate de guanéthidine, le chlorhydrate d'hydralazine, l'hydrochlorothiazide, l'hydrofluméthiazide, l'acide (+)-4-[3-[-[2-(1-hydroxycyclohexyl) éthyl]-4-oxo-2-thiazolidinyl]propyl]benzoïque, l'indacrinone et divers rapports de ses énantiomères, la méréthoxylline-procaïne, le méthylclothiazide, la méthyldopa, le chlorhydrate de méthyldopa, la métolazone, le tartrate de métoprolol, le minoxidil, le naldolol, la nifédipine, le chlorhydrate de pargyline, le pindolol, le polythiazide, la prazosine, le propranolol, la guinéthazone, Rauwolfia serpentina, la rescinnamine, la réserpine, l'éthacrynate de sodium, le nitroprussiate de sodium, la spironolactone, le ticrynafen, le timolol, le triamtérène, le trichlorméthiazide, le camsylate de triméthaphan, le bépridil, le dilthiazem, l'étafénone, le falipamil, la félodipine, la flunarizine, le gallopamil, l'indapamide, la lidoflazine, la nicardipine, la nifédipine, la nimopidine, la nitrendipine, la perhexiline, la prénylamine, le tiapamil, le vérapamil, ainsi que leurs mélanges et leurs combinaisons.

**5.** Composition pharmaceutique utile pour le traitement des troubles gastro-intestinaux, des troubles du

système nerveux central ou pour la régulation de l'appétit chez l'homme, comprenant une quantité pharmaceutiquement efficace d'un composé de la revendication 1 et un véhicule pharmaceutiquement acceptable.

**6.** Composition selon la revendication 5, dans laquelle ledit composé fait partie du groupe constitué par :
le 1-(3-acétoxy-2-oxopropyl)-3-(1-carboéthoxy-3-phényl-1-propyl) aminohomodihydrocarbostyryle ;
le 1-(3-hydroxy-2-oxopropyl)-3-(1-carboéthoxy-3-phényl-1-propyl) aminohomodihydrocarbostyryle ;
le 1-(3-acétoxy-2-oxopropyl)-3-[1-carboéthoxy-3-(3-indolyl)-1-propyl] aminohomodihydrocarbostyryle ;
le 1-(3-acétoxy-2-oxopropyl)-3-[1-N-(n)hexylcarboxamido-3-(3-indolyl)-1-propyl]-aminohomodihydrocarbostyryle ; et
le 1-(3-hydroxy-2-oxopropyl)-3-[1-N-(n)hexylcarboxamido-3-(3-indolyl)-1-propyl]-aminohomodihydrocarbostyryle.

## Patentansprüche

**1.** Eine Verbindung der Formel:

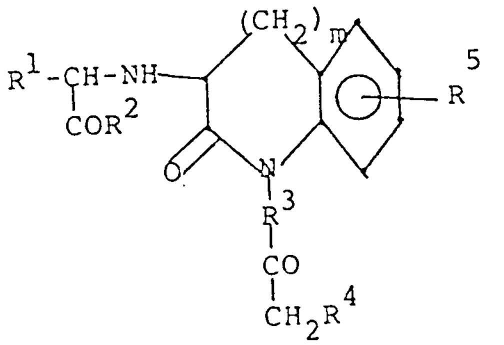

I

worin:

$R^1$ ist Wasserstoff;
Kohlenwasserstoff mit von 1 bis 12 Kohlenstoffatomen, der verzweigte und ungesättigte Alkylgruppen einschließt;
$C_3$-$C_{10}$-Cycloalkyl;
substituiertes $C_{1-6}$-Alkyl, worin der Substituent Halogen, Hydroxy, Carboxy, $C_{1-6}$-Alkylthio, $C_{1-6}$-Alkoxy, $C_{1-6}$-Alkoxycarbonyl, Carboxamido, $C_{1-6}$-Aralkoxycarbonyl, worin Aryl Phenyl, Naphthyl oder Biphenyl ist, Amino, $C_{1-6}$-Alkylamino, $C_{1-6}$-Dialkylamino oder $C_{1-6}$-Alkanoylamino und Aroylamino, worin Aryl Phenyl, Naphthyl oder Biphenyl ist, sein kann;
substituiertes Niedrigalkyl mit der Formel $R_A^1$ $(CH_2)_p$-Q-$(CH_2)_q$, worin p 0-2 ist, q 1-3 ist, $R_A^1$ Aryl oder Heteroaryl, worin Aryl Phenyl, Naphthyl oder Biphenyl ist und Heteroaryl ein 5- oder 6-gliedriger aromatischer Ring ist, der 1-3 Heteroatome enthält, ausgewählt aus der Gruppe bestehend aus Stickstoff, Sauerstoff und Schwefel, und bicyclische Gruppen ist, in denen die oben erwähnten 5- oder 6-gliedrigen aromatischen Ringe mit einem Benzolring kondensiert sind und worin die Aryl- oder Heteroarylgruppe gegebenenfalls substituiert ist durch Amino, $C_{1-6}$-Dialkylamino, $C_{1-6}$-Alkylamino, Hydroxy, Hydroxy-$C_{1-6}$-alkyl, Amino-$C_{1-6}$-alkyl, Trihalogen-$C_{1-6}$-alkyl, Cyano, Nitro, Sulfonamido, Aroyl, worin Aryl wie oben definiert ist, $C_{1-6}$-Alkyl, Halogen, Dihalogen und $C_{1-6}$-Alkoxy und worin Q O, S, N-$R_B^1$ CON$R_C^1$ , N$R_C^1$ CO, CH=CH,
worin $R_B^1$
Wasserstoff, $C_{1-6}$-Alkyl, Aryl wie oben definiert, Aralkyl, worin der Alkylteil $C_{1-6}$-Alkyl ist und der Arylteil wie oben definiert ist, $C_{1-6}$-Alkanoyl oder Aroyl, worin Aryl wie oben definiert ist, ist und worin $R_C^1$ Wasserstoff oder $C_{1-6}$-Alkyl ist;
Aryl wie oben definiert;
substituiertes Aryl wie oben definiert, worin der Substituent $C_{1-6}$-Alkyl, Amino-$C_{1-6}$-alkyl, $C_{1-6}$-Alkoxy, Aryloxy, worin Aryl wie oben definiert ist, Aroyl, worin Aryl wie oben definiert ist, Hydroxy, Halogen oder Dihalogen ist; substituiertes oder unsubstituiertes Aralkyl oder

substituiertes oder unsubstituiertes Heteroaralkyl, worin der Alkylteil $C_{1-6}$-Alkyl ist und der Arylteil und der Heteroarylteil wie oben definiert sind, die verzweigte $C_{1-6}$-Alkylgruppen einschliessen, worin die $C_{1-6}$-Alkylgruppen durch Amino, $C_{1-6}$-Alkanoylamino und Aroylamino, worin Aryl wie oben definiert ist, oder Hydroxyl substituiert sein können und worin die Aryl- und Heteroarylgruppen durch Halogen, Dihalogen, $C_{1-6}$-Alkyl, Hydroxy, $C_{1-6}$-Alkoxy, Aryloxy, worin Aryl wie oben definiert ist, Aroyl, worin Aryl wie oben definiert ist, Arylthio, worin Aryl wie oben definiert ist, Amino, Amino-$C_{1-6}$-Alkyl, $C_{1-6}$-Alkanoylamino, Aroylamino, worin Aryl wie oben definiert ist, $C_{1-6}$-Dialkylamino, $C_{1-6}$-Alkylamino, Hydroxy-$C_{1-6}$-alkyl, Trihalogen-$C_{1-6}$-alkyl, Nitro, Cyano oder Sulfonamido substituiert sein können,

$R^2$ ist Hydroxy; $C_{1-6}$-Alkoxy;
$C_{2-6}$-Alkenyloxy; Aryl-$C_{1-6}$-alkoxy, worin Aryl wie oben definiert ist; $C_{1-6}$-Alkanoyl-$C_{1-6}$-alkoxy; Amino; $C_{1-6}$-Alkylamino; Di-$C_{1-6}$-alkylamino; Aryl-$C_{1-6}$-alkylamino, worin Aryl wie oben definiert ist; Arylamino, worin Aryl wie oben definiert ist; Carboxy-$C_{1-6}$-alkylamino, Carboxamido-$C_{1-6}$-alkylamino oder Aryloxy oder mono- oder di-substituiertes Aryloxy, worin Aryl wie oben definiert ist und worin die Substituenten Halogen sind;

m 1-3 ist;

$R^3$ $C_{1-6}$-Alkyl oder substituiertes $C_{1-6}$-Alkyl ist, worin die Substituenten Wasserstoff, $C_{1-6}$-Alkyl oder Aryl wie oben definiert sind;

$R^4$ Hydroxy; $C_{1-6}$-Alkanoyloxy; Aryl-$C_{1-6}$-alkanoyloxy ist, worin Aryl wie oben definiert ist;

$R^5$ Wasserstoff; Halogen; Hydroxy; $C_{1-6}$-Alkyl oder $C_{1-6}$-Alkoxy ist; und

ein pharmazeutisch annehmbares Salz davon.

**2.** Eine Verbindung nach Anspruch 1, die ein Glied der Gruppe:
1-(3-Acetoxy-2-oxopropyl)-3-(1(S)-carboethoxy-3-phenyl-1-propyl)-(S)-aminohomodihydrocarbostyril;
1-(3-Hydroxy-2-oxopropyl)-3-(1(S)-carboethoxy-3-phenyl-1-propyl)-(S)-aminohomodihydrocarbostyril;
und
1-(3-Hydroxy-2-oxopropyl)-3-(1(S)-carboxy-3-phenyl-1-propyl)-(S)-aminohomodihydrocarbostyril ist.

**3.** Eine für die Behandlung von Hypertonie brauchbare pharmazeutische Zusammensetzung, umfassend einen pharmazeutisch annehmbaren Träger und eine antihypertonisch wirksame Menge einer Verbindung nach Anspruch 1.

**4.** Eine Zusammensetzung nach Anspruch 3, die eine antihypertonische und/oder diuretische und/oder eine den Calciumeintritt blockierende Verbindung einschließt, ausgewählt aus der Gruppe bestehend aus Acetazolamid, Amilorid, Aminophyllin, Atenolol, Bendroflumethiazid, Benzthiazid, Bumetanid, Chlorthalidon, Chlorothiazid, Clonidin, Cryptenaminacetate und Cryptenamintanate, Cyclothiazid, Deserpidin, Diazoxid, Diltiazem, (S)-1-[[2-(3,4-Dimethoxyphenyl)-ethyl]-amino]-3-[[4-(2-thienyl)-1H-imidazol-2-yl]-phenoxy]-2-propanol, Etacrynsäure, Flumethiazid, Furosemid, Guanethidensulfat, Hydralazinhydrochlorid, Hydrochlorothiazid, Hydroflumethiazid, (+)-4-[3-[-[2-(1-Hydroxycyclohexyl)-ethyl]4-oxo-2-thiazolidinyl]propyl]-benzoesäure, Indacrinon und variablen Verhältnissen seiner Enantiomere, Merethoxyllin-Procain, Methylclothiazid, Methyldopa, Methyldopathydrochlorid, Metolazon, Metoprololtartrat, Minoxidil, Naldolol, Nifedipin, Pargylinhydrochlorid, Pindolol, Polythiazid, Prazosin, Propranolol, Quinethazon, Rauwolfia serpentina, Rescinnamin, Reserpin, Natriumetacrynat, Nitroprussidnatrium, Spironolacton, Ticrynafen, Timolol, Triamteren, Trichlormethiazid, Trimethophan-Camsilat, Bepridil, Diltiazim, Etafenon, Falipamil, Felodipin, Flunarizin, Gallopamil, Indapamid, Lidoflazin, Nicardipin, Nifedipin, Nimopidin, Nitrendipin, Perhexilin, Prenylamin, Tiapamil, Verapamil ebenso wie Beimischungen und Kombinationen davon.

**5.** Eine für die Behandlung von gastrointestinalen Störungen, von Störungen des zentralen Nervensystems oder zur Regulierung des Appetits bei Menschen brauchbare pharmazeutische Zusammensetzung, enthaltend eine pharmazeutisch wirksame Menge einer Verbindung nach Anspruch 1 und einen annehmbaren pharmazeutischen Träger.

**6.** Die Zusammensetzung nach Anspruch 5, worin die Verbindung ein Glied aus der Gruppe
1-(3-Acetoxy-2-oxopropyl)-3-(1-carboethoxy-3-phenyl 1-propyl)aminohomodihydrocarbostyril;
1-(3-Hydroxy-2-oxopropyl)-3-(1-carboethoxy-3-phenyl 1-propyl)aminohomodihydrocarbostyril;
1-(3-Acetoxy-2-oxopropyl)-3-[1-carboethoxy-3-(3-indolyl)-1-propyl]aminohomodihydrocarbostyril;

1-(3-Acetoxy-2-oxopropyl)-3-[1-N(n)hexylcarboxamido-3-(3-indolyl)-1-propyl]-aminohomodihydrocarbostyril; und
1-(3-Hydroxy-2-oxopropyl)-3-[1-N(n)hexylcarboxamido-3-(3-indolyl)-1-propyl]-aminohomodihydrocarbostyril ist.